# EUROPÄISCHE PATENTSCHRIFT

(11) **EP 4 101 486 B1**
(45) Veröffentlichungstag und Bekanntmachung des Hinweises auf die Patenterteilung: **18.02.2026**
(21) Anmeldenummer: 21178968.0
(22) Anmeldetag: 11.06.2021
(51) Int. Cl.: A61M 11/00, B05B 1/14, B05B 11/02, A61M 15/00, A61M 15/08, B05B 11/10

(54) **FLÜSSIGKEITSSPENDER FÜR NASALE ANWENDUNGEN**
LIQUID DISPENSER FOR NASAL APPLICATIONS
DISTRIBUTEUR DE LIQUIDE POUR APPLICATIONS NASALES

(43) Veröffentlichungstag der Anmeldung: 14.12.2022
(73) Patentinhaber: Aptar Radolfzell GmbH, 78315 Radolfzell (DE)
(72) Erfinder: Greiner-Perth, Jürgen, 78244 Gottmadingen (DE); Ritsche, Stefan, 78253 Eigeltingen (DE)
(74) Vertreter: Witte, Weller und Partner Patentanwälte mbB Stuttgart

(56) Entgegenhaltungen:
- WO-A2-2020/216599
- DE-A1- 102018 220 632
- US-A1- 2003 158 527
- US-A1- 2008 081 079
- US-A1- 2009 008 415
- US-B2- 11 007 332

## Beschreibung

### ANWENDUNGSGEBIET UND STAND DER TECHNIK

Die Erfindung betrifft einen Flüssigkeitsspender für nasale Anwendungen. Ein solcher Spender dient dem Zweck, pharmazeutische Flüssigkeiten in die Nase und die Atemwege eines Patienten einzubringen.

Ein gattungsgemäßer Flüssigkeitsspender verfügt über einen Flüssigkeitsspeicher, in dem die pharmazeutische Flüssigkeit vor dem Austrag gelagert ist, sowie über einen länglichen Nasalapplikator, an dessen distalem Ende mindestens eine Austragöffnung vorgesehen ist, durch die die Flüssigkeit ausgetragen wird.

Um Flüssigkeit auszutragen, ist bei gattungsgemäßen Flüssigkeitsspendern eine Betätigung vorgesehen, die insbesondere bei Abgabe der Flüssigkeit in Tropfenform über ein Zusammendrücken des Flüssigkeitsspeichers erzielt wird und insbesondere bei Abgabe der Flüssigkeit in zerstäubter Form durch eine Betätigungshandhabe erfolgt, die mittelbar eine Druckbeaufschlagung der Flüssigkeit bewirkt.

Die vorliegende Erfindung betrifft Flüssigkeitsspender, bei denen die Flüssigkeit im Zuge des Austrags zerstäubt wird, also in einen Nebel aus sehr kleinen Flüssigkeitstropfen überführt wird. Dies erfolgt bei den meisten gattungsgemäßen Spendern über eine der Austragöffnung vorgeschaltete Wirbelkammer, in die die Flüssigkeit tangential eingeleitet wird. Durch die erzielte schnelle rotierende Bewegung wird die Flüssigkeit beim Austritt in feine Tropfen zerrissen. Das Dokument US 2008/081079 A1 stellt relevanten Stand der Technik dar.

### AUFGABE UND LÖSUNG

Aufgabe der Erfindung ist es, einen Flüssigkeitsspender für nasale Anwendungen zur Verfügung zu stellen, der keine Wirbelkammer benötigt und eine besonders feine Zerstäubung der Flüssigkeit gestattet.

Ein erfindungsgemäßer Flüssigkeitsspender weist an einem distalen Ende des Nasalapplikators eine Düsenplatte mit einer Vielzahl von Düsenöffnungen auf. Die Düsenplatte weist mindestens 25 Düsenöffnungen auf, deren mittlere lichte Querschnittsfläche an der engsten Stelle jeweils maximal 500 µm² beträgt. Insbesondere bevorzugt sind jedoch Gestaltungen mit erheblich mehr Düsenöffnungen, die vorzugsweise erheblich kleiner gestaltet sind, wie im Weiteren noch beschrieben ist.

Durch die Düsenöffnungen wird die Flüssigkeit in der Form einer Vielzahl feiner Strahlen abgegeben, die jenseits der Düsenöffnungen zerfallen ("Rayleigh breakup").

Aufgrund der kleinen Düsenöffnungen, die benötigt werden, um den Zerfall des Strahls in Einzeltropfen zu erreichen, stellt es jedoch ein Problem dar, eine ausreichende Flüssigkeitsmenge auszutragen. Die üblicherweise bei einem Austrag zu erzielende Flüssigkeitsmenge beträgt üblicherweise zwischen 50 µl und 150 µl. Von anderen Spendern bekannte Düsenplatten gestatten meist nur den Austrag eines Flüssigkeitsstroms von weniger als 50 µl / Sekunde, so dass der Austrag der genannten Flüssigkeitsmenge drei Sekunden und mehr dauern kann. Es hat sich in der Praxis jedoch gezeigt, dass ein Betätigungsvorgang von solcher Dauer für den Nutzer ungewohnt ist und dies zu einem zu frühen Abbruch der Betätigung führen kann.

Um den Austrag einer solchen Flüssigkeitsmenge zuverlässig erfolgen zu lassen, wird vorgeschlagen, dass der Flüssigkeitsspender über eine Fördereinrichtung zur Förderung der Flüssigkeit aus dem Flüssigkeitsspeicher zu den Düsenöffnungen verfügt, welche ein bewegliches Druckelement aufweist, das in Art eines Kolbens oder einer anderweitigen verlagerbaren Wandung der Druckbeaufschlagung der Flüssigkeit dient.

Zur Verlagerung dieses Druckelements weist die Fördereinrichtung eine Betätigungshandhabe zur manuellen Verlagerung auf, die im Rahmen der Betätigung aus einer Ausgangslage in eine betätigte Endlage verlagert wird. Dabei ist vorgesehen, dass die stattfindende Verlagerung der Betätigungshandhabe nicht unmittelbar auf das Druckelement übertragen wird. Stattdessen sind die Betätigungshandhabe und das Druckelement über einen Federspeicher verbunden, welcher durch Verlagerung der Betätigungshandhabe in die Endlage geladen wird und welcher anschließend das Druckelement bewegt, nachdem die Betätigungshandhabe bereits die Endlage erreicht hat.

Die Verlagerung des Betätigungselements führt also bestimmungsgemäß zu keiner oder nur zu einer geringen gleichzeitigen Verlagerung des Druckelements. Stattdessen wird das Federelement, welches beispielsweise in Form einer insbesondere metallischen Schraubenfeder oder Schenkelfeder ausgestaltet sein kann, gespannt. Die Bewegung des Druckelements wird maßgeblich durch dieses sich nach dem Spannen wieder entspannende Federelement angetrieben, nachdem die Betätigungshandhabe bereits ihre Endlage erreicht hat.

Der Flüssigkeitsspender ist vorzugsweise derart abgestimmt, dass bei gedachter schlagartiger Verlagerung der Betätigungshandhabe in die Endlage und somit schlagartigem Spannen des Federspeichers der anschließend durch den Federspeicher bewirkte Austrag von Flüssigkeit mindestens 0,3 Sekunden dauert, vorzugsweise mindestens 0,5 Sekunden, insbesondere vorzugsweise mindestens 1 Sekunde. Grundsätzlich können jedoch auch wesentlich längere Zeiträume vorgesehen sein, beispielsweise von mindestens zwei Sekunden oder gar von mindestens drei Sekunden.

Maßgeblich für den Austragzeitraum ist vor allem der Strömungswiderstand, der durch die Düsenöffnungen erzeugt wird, sowie der durch den Federspeicher bewirkte Flüssigkeitsdruck.

Der genannten Zeiträume von beispielsweise drei Sekunde sind in der Praxis zu lang, um vom Patienten zu erwarten, dass dieser über einen solchen Zeitraum die Betätigungshandhabe kontinuierlich kraftbeaufschlagt. Durch den erfindungsgemäß vorgesehenen Federspeicher kann der Patient hiervon entbunden werden, da er die Betätigungshandhabe direkt bis in die Endlage drücken kann und dabei jene Energie in das System einbringt, die anschließend fortgesetzt die Druckbeaufschlagung der Flüssigkeit bewirkt. Wäre da Federelement nicht vorhanden und die Betätigungshandhabe direkt mit dem Druckelement verbunden, so wäre eine unmittelbare und schnelle Verlagerung der Betätigungshandhabe in die Endlage nicht möglich, da der Flüssigkeitsaustrag durch die Düsenöffnungen auch bei deutlich erhöhtem Druck nicht ausreichend schnell Flüssigkeit austragen können.

Ein Flüssigkeitsspender der beschriebenen Art kann insbesondere in zweierlei Weise ausgebildet sein.

Bei einer ersten Variante begrenzt das Druckelement den Flüssigkeitsspeicher unmittelbar. Der durch das Druckelement erzeugte Druck führt also zu Druckbeaufschlagung der gesamten Flüssigkeit. Insbesondere kann bei einer solchen Variante vorgesehen sein, dass die Flüssigkeit im Flüssigkeitsspeicher im Rahmen einer einzigen Betätigung oder im Rahmen zweier Betätigungen ausgetragen wird. Entsprechende Spender werden auch als Unitdose-Spender oder Bidose-Spender bezeichnet. Bei einer solchen Gestaltung ist das Druckelement vorzugsweise Teil eines Bauteils, welches in Art eines einseitig geschlossenen Zylinders gestaltet ist, der als Ganzes gegenüber einem zum Nasalapplikator ortsfesten Kolben verlagert wird.

Bei einer zweiten Variante begrenzt das Druckelement eine Pumpkammer, insbesondere in Art eines in der Pumpkammer verlagerbaren Kolbens. Diese Pumpkammer ist Teil einer Pumpeinrichtung. Sie ist über einen Eingangskanal mit Einlassventil mit dem Flüssigkeitsspeicher verbunden sowie über einen Ausgangskanal mit Auslassventil mit den Düsenöffnungen verbunden. Die mittelbar über die Betätigungshandhabe und unmittelbar über den Federspeicher angetriebene Verlagerung des Druckelements führt dementsprechend bei dieser Variante nicht zu einer Druckbeaufschlagung der Flüssigkeit im Flüssigkeitsspeicher, sondern nur zu einer Druckbeaufschlagung der Flüssigkeit in der Pumpkammer und stromabwärts hiervon.

Im einfachsten Falle kann bei einem Flüssigkeitsspender der beschriebenen Art vorgesehen sein, dass der Patient die Betätigungshabe in die Endlage drückt und gedrückt hält, bis der Federspeicher sich entspannt hat und der Austrag endet.

Da dies jedoch die Gefahr einer Fehlbedienung durch zu frühes Beenden der Kraftbeaufschlagung birgt, wird es als bevorzugt angesehen, wenn eine selbsttätig einrastende Rasteinrichtung an einem zum Nasalapplikator ortsfesten Gehäuseabschnitt vorgesehen ist, mittels derer die Betätigungshandhabe in ihrer Endlage gesichert wird. Eine solche Rasteinrichtung umfasst insbesondere vorzugsweise ein elastisch auslenkbares Rastelement mit einer Schräge, welches durch die Betätigungshabe oder ein gemeinsam mit der Betätigungshabe bewegtes Teilelement bei der Betätigung ausgelenkt wird und bei Erreichen der Endlage unter dem Eindruck der elastischen Auslenkung mit der Betätigungshabe oder dem genannten Teilelement einrastet. Statt an einem Gehäuseabschnitt kann die auslenkbare Rasteinrichtung auch an der Betätigungshandhabe oder einem hiermit bewegten anderen Teilelement vorgesehen sein.

Die Rasteinrichtung bewirkt, dass der Patient nach Betätigung der Betätigungshandhabe unmittelbar die Kraftbeaufschlagung beenden kann, da die Endlage nun durch die Rasteinrichtung gehalten wird. Die Rasteinrichtung ist darüber hinaus von Vorteil, da sie dem Patienten durch ein hörbares oder spürbares Einrasten verdeutlicht, dass er die Endlage erreicht hat.

Insbesondere bei einem Spender, der zur einmaligen Verwendung vorgesehen ist, ist diese Rasteinrichtung vorzugsweise als unlösbare Rasteinrichtung vorgesehen. Dies bedeutet, dass der Patient keine bestimmungsgemäße Möglichkeit hat, die Verrastung wieder zu lösen. Insbesondere kann die Rasteinrichtung an einen für den Patienten unzugänglichen Ort des Spenders angeordnet sein, insbesondere geschützt von umgebenden Wandungen des Spenders.

Alternativ kann die Rasteinrichtung als bestimmungemäß lösbare Rasteinrichtung ausgebildet sein. Dies ist insbesondere bei der oben beschriebenen Gestaltung des Flüssigkeitsspenders mit einer Pumpeinrichtung zweckmäßig, da hier die Betätigungshandhabe für eine weitere Betätigung in ihre Ausgangslage zurückkehren können muss.

Bei einer lösbaren Rasteinrichtung ist vorzugsweise eine separate Freigabetaste vorgesehen, durch die die Verrastung gelöst wird. Insbesondere kann das oben beschriebene elastisch auslenkbare Rastelement durch diese Freigabetaste verlagert werden, so dass es die Betätigungshandhabe freigibt. Unter dem Eindruck des Federspeichers und/oder eine separaten Kolbenfeder der Pumpe wird die Betätigungshandhabe dann zurück in die Ausgangslage gedrückt wird.

Zwei Bauweisen eines erfindungsgemäßen Spenders werden bevorzugt. Bei der ersten dieser Bauweisen ist vorgesehen, dass die Betätigungshandhabe translativ in Richtung einer Haupterstreckungsrichtung des Nasalapplikators und/oder einer Hauptaustragsrichtung verlagerbar ist, wobei die Haupterstreckungsrichtung und die Hauptaustragrichtung vorzugweise identisch sind. Insbesondere vorteilhaft ist es, wenn die Betätigungshandhabe gegenüberliegend zum Nasalapplikator am Spender vorgesehen ist und nach oben in Richtung des Nasalapplikators gedrückt wird.

Vorzugsweise ist zur bequemen Bedienung mindestens eine ortsfest zum Nasalapplikator angebrachte Gegenkraftfläche zur manuellen Stützung vorgesehen. Insbesondere kann es sich um zwei Gegenkraftflächen handeln, die beidseitig des Nasalapplikators vorgesehen sind. Auf diese beiden Gegenkraftflächen werden bestimmungsgemäß der Zeigefinger und der Mittelfinger aufgelegt, während der Daumen auf der Betätigungshandhabe aufgelegt wird und diese im Zuge der Betätigung in Richtung der Gegenkraftflächen drückt.

Die zweite besonders bevorzugte Bauweise sieht vor, dass der Flüssigkeitsspender ein in Richtung einer Haupterstreckungsrichtung ausgerichtetes längliches Gehäuse aufweist, an dessen distalem Ende die Düsenöffnungen vorgesehen ist und an dessen gegenüberliegendem Ende vorzugsweise der Flüssigkeitsspeicher vorgesehen ist. Die Betätigungshandhabe ist bei dieser Bauweise seitlich am Gehäuse und somit exzentrisch zu einer Mittelachse angeordnet. Die Betätigung der Betätigungshandhabe erfolgt in Richtung der Mittelachse, wobei sowohl eine schwenkbewegliche Betätigung als auch eine translative Betätigung vorgesehen sein können. Ein solcher Spender mit seitlich angebrachter Betätigungshandhabe wird auch als Side-Actuation-Spender bezeichnet.

Die genannte zweite Bauweise ist insbesondere bei einem Spender mit einer Pumpeinrichtung zweckmäßig. Insbesondere vorzugsweise ist dabei vorgesehen, dass das Druckelement orthogonal zur Haupterstreckungsachse des Spenders verlagerbar ist, die Pumpeinrichtung also quer in das Gehäuse eingebaut ist. Allerdings sind auch Gestaltungen denkbar, bei denen zwischen der Betätigungshandhabe und dem Druckelement weitere Getriebeelemente vorgesehen sind, die einer Umsetzung der Kraft oder der Kraftrichtung dienen, so dass auch eine in Haupterstreckungsrichtung ausgerichtete Pumpeinrichtung möglich ist.

Die eingangs beschriebene Problematik, dass die feinen Düsenöffnungen einen zeitlich langen Austrag erforderlich machen, kann auch durch einen Flüssigkeitsspender begegnet werden, der in oben beschriebener Weise als Flüssigkeitsspender für nasale Anwendungen ausgebildet ist und einen Flüssigkeitsspeicher sowie einen Nasalapplikator mit Düsenplatte aufweist, bei dem jedoch die zusätzliche Besonderheit gegeben ist, dass die Düsenplatte für einen vergleichsweise großen Flüssigkeitsstrom ausgelegt ist.

Erfindungsgemäß ist dabei vorgesehen, dass die Gesamtheit der Düsenöffnungen derart ausgelegt ist, dass ein Gesamtflüssigkeitsstrom vom mindestens 100 µl / Sekunde durch die Düsenöffnungen erzielt wird, wenn Flüssigkeit mit den Eigenschaften von Wasser in einer der Düsenplatte vorgeschalteten Vorkammer unter einem Druck von 5 bar an der Düsenplatte anliegt.

Bei Verwendung einer solchen Düsenplatte kann die Austragmenge von 50 µl bis 150 µl, die üblicherweise beim Austrag von pharmazeutischen Flüssigkeiten mittels Nasalapplikatoren je Austragvorgang vorgesehen ist, in 1,5 Sekunden oder weniger ausgetragen werden.

Wenngleich in der oben beschriebenen Weise ein Flüssigkeitsspender von Vorteil ist, der über einen Federspeicher und insbesondere auch eine Rasteinrichtung der beschriebenen Art verfügt, kann bei Verwendung einer solchen für großen Flüssigkeitsstrom ausgelegten Düsenplatte und somit bei einer Austragdauer von vorzugsweise 1,5 Sekunden oder weniger auf das Federelement und die Rasteinrichtung auch verzichtet werden und stattdessen vorgesehen sein, dass der Patient über die Betätigungshandhabe unmittelbar das Druckelement verlagert und somit die Druckbeaufschlagung der Flüssigkeit bewirkt. Bevorzugt ist allerdings die Nutzung eines Spenders der oben beschriebenen Art mit Federspeicher.

Besonders bevorzugt sind Gestaltungen der Düsenplatte, die für einen Gesamtflüssigkeitsstrom zwischen 100 µl / Sekunde und 750 µl / Sekunde bei 5 bar Flüssigkeitsdruck ausgelegt sind, insbesondere vorzugsweise für einen Gesamtflüssigkeitsstrom zwischen 200 µl / Sekunde und 500 µl / Sekunde.

Zur Erzielung solcher recht großer Flüssigkeitsströme sind verschiedene Möglichkeiten gegeben. Als insbesondere bevorzugenswert hat sicher eine Gestaltung herausgestellt, bei der zwischen 400 und 2000 Düsenöffnungen vorgesehen sind. Die Düsenöffnungen weisen dabei jeweils eine minimale lichte Querschnittsfläche zwischen 10 µm² und 20 µm² auf, vorzugsweise zwischen 10 µm² und 15 µm². Vorzugsweise sind die Düsenöffnungen rund ausgebildet und weisen einen entsprechenden minimalen Durchmesser auf, insbesondere zwischen 1,5 µm und 3 µm.

Die Herstellung derart kleiner Düsenöffnungen ist technisch aufwändig, so dass dies auch den Preis des Flüssigkeitsspenders erhöht.

Es hat sich jedoch gezeigt, dass auch mit einer Düsenplatte mit 200 bis 1000 Düsenöffnungen gute Ergebnisse zu erzielen sind. Hierbei sind die Düsenöffnungen etwas größer gestaltet und weisen eine minimale lichte Querschnittsfläche zwischen 20 µm² und 50 µm² auf, vorzugsweise zwischen 25 µm² und 35 µm². Im Falle von runden Düsenöffnungen liegt deren Durchmesser im Bereich zwischen 2,5 µm und 4 µm.

Nochmals vereinfacht werden kann die Herstellung, indem eine noch geringere Zahl von Düsenöffnungen vorgesehen ist, nämlich zwischen 50 und 250 Düsenöffnungen, die jeweils eine minimale lichte Querschnittsfläche zwischen 50 µm² und 100 µm² aufweisen, vorzugsweise zwischen 70 µm² und 85 µm². Im Falle von runden Düsenöffnungen liegt deren Durchmesser im Bereich zwischen 4 µm und 6 µm.

Diese vergleichsweise großen Düsenöffnungen sind in Hinblick auf die Zerstäubung nicht ideal. Allerdings erlauben sie es, bei geringen Herstellungskosten den genannten hohen Gesamtflüssigkeitsstrom vom mindestens 100 µl / Sekunde zu erzielen. Für preisgünstige Einwegspender kann diese Auslegung daher sinnvoll sein.

Vorzugsweise sind die oben genannten Querschnittsflächen für alle Düsenöffnungen gleich. Sofern die Düsenöffnungen bezüglich ihrer lichten Querschnittsfläche unterschiedlich gestaltet sind, wird es als bevorzugt angesehen, wenn mindestens 80% der Düsenöffnungen die jeweils genannten minimalen Querschnittsflächen aufweisen.

Die Herstellung der Düsenplatte kann auf verschiedenen Wegen erfolgen. Eine besonders bevorzugte Gestaltung sieht vor, dass die Düsenplatten als metallische Düsenplatten ausgebildetsind und per Galvanoformen hergestellt werden. Hierbei wird Metall, bspw. Kupfer oder Nickel, elektrolytisch aus einem wässrigen Salzbad abgeschieden, so dass in additiver Art und Weise die Düsenplatte aufgebaut wird.

Eine alternative Technik sieht vor, dass die Düsenöffnungen per Laser in einen Rohling eingebracht werden. Der Rohling der Düsenplatte kann dabei als dünne metallische Düsenplatte, keramische Düsenplatte, Düsenplatte aus Glas oder einem anderen mineralischen Werkstoff oder Düsenplatte aus Kunststoff hergestellt sein, in die anschließend über einen Laserstrahl oder vorzugsweise viele Laserstrahlen die Düsenöffnungen eingebracht worden sind.

Eine dritte Möglichkeit besteht darin, die Düsenplatte aus Kunststoff, insbesondere aus Polypropylen, per Spritzguss herzustellen und dabei durch die Formgebung der verwendeten Kavität unmittelbar die Düsenöffnungen von Material freizuhalten.

Aufgrund der hohen Zahl von Düsenöffnungen ist es von besonderer Bedeutung, zu verhindern, dass die austretenden Flüssigkeitsstrahlen in Kontakt miteinander gelangen und dadurch eine feine Zerstäubung unterbunden wird. Die Düsenöffnungen sind daher vorzugweise in divergierender Ausrichtung vorgesehen, wobei außenliegende Düsenöffnungen stärke gegenüber einer mittleren Austragrichtung angewinkelt sind als innenliegende Düsenöffnungen. Bei Herstellung der Düsenöffnungen per Laser oder per Galvanoformen kann dies unmittelbar in der ebenen Düsenplatte realisiert sein. Es kann insbesondere jedoch auch erzielt werden, indem die Düsenplatte im Flüssigkeitsspender in gewölbter Formgebung eingesetzt ist. Eine solche Wölbung kann beispielsweise durch einen ringförmigen Kunststoffträger erzwungen werden, in den die Düsenplatte eingesetzt ist.

### KURZBESCHREIBUNG DER ZEICHNUNGEN

Weitere Vorteile und Aspekte der Erfindung ergeben sich aus den Ansprüchen und aus der nachfolgenden Beschreibung von bevorzugten Ausführungsbeispielen der Erfindung, die nachfolgend anhand der Figuren erläutert sind.
Fig. 1 zeigt ein erstes Ausführungsbeispiel eines erfindungsgemäßen Spenders.
Fig. 2 bis 4 zeigen die Düseneinheit des Spenders der Fig. 1 sowie dessen Düsenplatte in einer geschnittenen Ansicht sowie in einer Draufsicht.
Fig. 5A bis 5D zeigen den Ablauf der Betätigung des Spenders der Fig. 1.
Fig. 6A bis 6D zeigen ein zweites Ausführungsbeispiel eines erfindungsgemäßen Spenders sowie den Ablauf der Betätigung dieses Spenders.

### DETAILLIERTE BESCHREIBUNG DER AUSFÜHRUNGSBEISPIELE

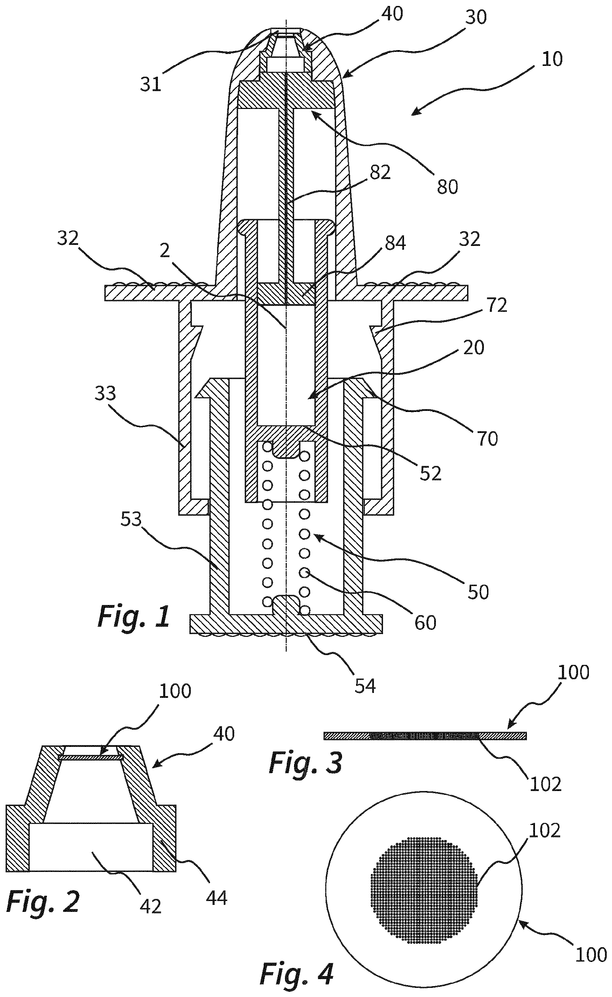

Fig. 1 zeigt einen erfindungsgemäßen Flüssigkeitsspender 10 gemäß einem ersten Ausführungsbeispiel.

Der Flüssigkeitsspender 10 ist als Flüssigkeitsspender für nasale Anwendungen vorgesehen und weist zu diesem Zweck einen Nasalapplikator 30 auf, an dessen distalem Ende eine Öffnung 31 vorgesehen ist, durch die Flüssigkeit abgegeben werden kann. Der Nasalapplikator 30 wird außenseitig durch ein Gehäusebauteil gebildet, welches noch weitere Teilbestandteile aufweist, die im Weiteren erläutert sind.

Zum Zwecke des Flüssigkeitsaustrags ist eine Fördereinrichtung 50 vorgesehen, die über eine hülsenförmige Betätigungseinheit 53 verfügt, welche von unten in einen zum Nasalapplikator ortsfesten Führungsschacht 33 eingeschoben ist. Die Unterseite der Betätigungseinheit 53 wird durch eine Betätigungshandhabe 54 gebildet. Gegenkraftflächen 32 hierzu sind beidseitig vom Nasalapplikator 30 abragend vorgesehen.

An der Öffnung 31 am distalen Ende des Nasalapplikator 30 ist eine Düseneinheit 40 vorgesehen, die in Fig. 2 genauer dargestellt ist. Die Düseneinheit 40 verfügt über eine Kunststoffhülse 44, in die eine Düsenplatte 100 eingesetzt ist. Stromaufwärts der Düsenplatte 100 ist eine Vorkammer 42 vorgesehen, in der sich druckbeaufschlagte Flüssigkeit sammelt, um dann durch Düsenöffnungen 102 der Düsenplatte 100 hindurch ausgetragen zu werden.

Wieder bezugnehmend auf Fig. 1 ist stromaufwärts der Düseneinheit 40 ein Innenbauteil 80 in den Nasalapplikator 30 eingesetzt, welches von einem Austragkanal 82 durchdrungen wird. Das gegenüberliegende Ende des Innenbauteils 80 ist in Art eines Kolbens 84 ausgestaltet, der mit seiner Außenseite gleitend und dichtend an der Innenseite eines zylindrischen Flüssigkeitsspeichers 20 anliegt, dessen Boden durch ein Druckelement 52 gebildet wird
Der Flüssigkeitsspeicher 20 ist gegenüber dem Innenbauteil 80 und dem Nasalapplikator axial verlagerbar, wobei die bereits beschriebene Betätigungseinheit 53 zur Verlagerung des Flüssigkeitsspeichers 20 und des Druckelements 52 vorgesehen ist.

Allerdings sind die Betätigungseinheit 53 einerseits und der Flüssigkeitsspeicher 20 mit Druckelement 52 andererseits nicht unmittelbar miteinander verbunden, sondern stattdessen über einen Federspeicher 60 in Form einer Schraubenfeder gekoppelt.

Die Fig. 3 und 4 zeigen die Düsenplatte 100, durch die hindurch der Austrag der Flüssigkeit erfolgt. Die Düsenplatte kann beispielsweise aus Metall oder Keramik bestehen. Es sind knapp 1200 DüsenÖffnungen 102 vorgesehen, die die Düsenplatte durchdringen und die jeweils eine minimale lichte Querschnittsfläche von etwa 10 µm² aufweisen. Gemeinsam gestatten die Düsenöffnungen bei einem stromaufwärts anliegenden Flüssigkeitsdruck von 5 bar einen Flüssigkeitsstrom von mehr als 100 µ / Sekunde.

Die Düsenöffnungen 102 können zueinander parallel ausgerichtet sein. Vorzugsweise ist jedoch vorgesehen, dass die Düsenöffnungen 102 divergierend ausgerichtet sind, wobei die außenliegenden Düsenöffnungen 102 stärker von der Haupterstreckungsrichtung 2 divergieren als die innenliegenden Düsenöffnungen 102. Hierdurch wird gewährleistet, dass der Zerfall der austretenden Flüssigkeitsstrahlen in Einzeltröpfen in der gewünschten Weise stattfindet.

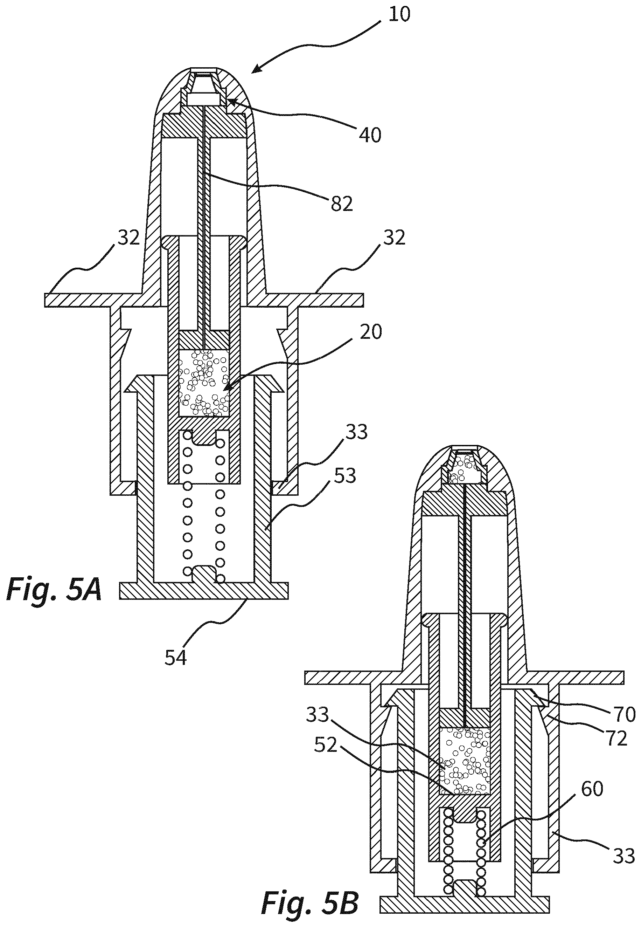

Anhand der Fig. 5A bis 5D wird die Handhabung des Flüssigkeitsspenders der Fig. 1 erläutert.

Fig. 5A zeigt einen Ausgangszustand des Flüssigkeitsspenders 10, der dem Zustand der Fig. 1 entspricht. Der Flüssigkeitsspeicher 20 ist mit einer pharmazeutischen Flüssigkeit befüllt, welche durch den Austragkanal 82 bis zur Düseneinheit 40 gelangen kann. In nicht dargestellter Art und Weise kann im Ausgangszustand jedoch vorgesehen sein, dass der Austragkanal 82 noch verschlossen ist, beispielsweise durch eine Membran, welche unter dem Eindruck von Flüssigkeitsdruck bestimmungsgemäß zerstört werden kann. Im Ausgangszustand der Fig. 5A ist vorzugsweise in nicht näher dargestellter Weise vorgesehen, dass die Betätigungseinheit 53 und das untere Ende des Schachtes 33 miteinander verrastet sind oder durch dünne Kunststoffbrücken miteinander verbunden sind, so dass ein unbeabsichtigtes Eindrücken der Betätigungseinheit 53 unterbunden wird und ein gewolltes Eindrücken mit einem fühlbaren Druckpunkt einhergeht

Ausgehend vom Zustand der Fig. 5A ergreift der Patient den Flüssigkeitsspender 10 derart, dass sein Zeigefinger und sein Mittelfinger auf den Gegenkraftflächen 32 platziert werden, während er den Daumen auf die Betätigungshandhabe 54 der Betätigungseinheit 53 legt. Ausgehend hiervon wird die Betätigungseinheit 53 mittels des Daumens nach oben kraftbeaufschlagt, wobei die oben genannte Verrastung gelöst oder die oben genannten Kunststoffbrücken zerbrochen werden.

Über den Federspeicher 60 wird die Betätigungskraft auch unmittelbar auf das Druckelement 52 und den Flüssigkeitsspeicher 20 übertragen. Dabei wird jedoch ein Großteil der eingebrachten Energie zunächst durch Kompression und damit Spannen des Federspeichers 60 aufgenommen. Ein sofortiger Austrag der Flüssigkeit aus dem Flüssigkeitsspeicher 20 findet nur in sehr begrenztem Maße statt, da die Düsenplatte 100 nur den genannten geringen Flüssigkeitsstrom gestattet.

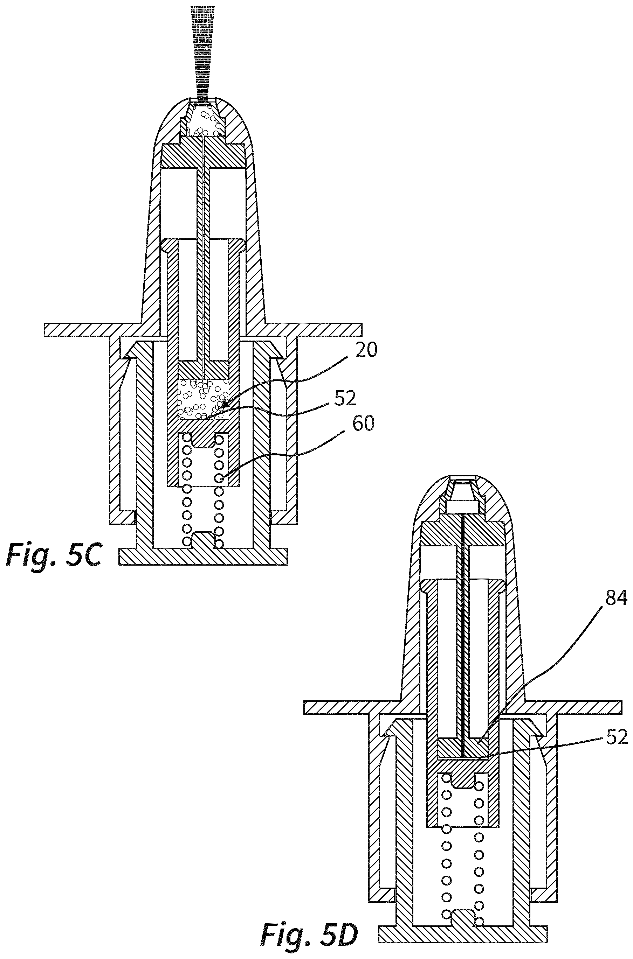

Ist die Betätigungseinheit 53 vollständig eingedrückt, so verrastet sie durch Rastelemente 70, 72 mit einer Innenseite des Schachtes 33. Dieser Zustand ist in Fig. 5B dargestellt. Durch die Verrastung verbleibt die Betätigungseinheit 53 im eingedrückten Zustand. Der unter starker Spannung stehende Federspeicher 60 drückt weiterhin auf das Druckelement 52 und den Flüssigkeitsspeicher 20, so dass das Volumen des Flüssigkeitsspeichers 20 verringert wird und über die nächsten beispielsweise 1 bis 2 Sekunden Flüssigkeit ausgetragen wird, wie in Fig. 5C verdeutlicht.

Erst wenn der Flüssigkeitsspeicher 20 so weit nach oben verschoben wurde, dass das kolbenartige Ende 84 des Innenbauteils 80 am Boden des Flüssigkeitsspeichers anliegt, endet der Flüssigkeitsaustrag. Dies ist in Fig. 5D verdeutlicht.

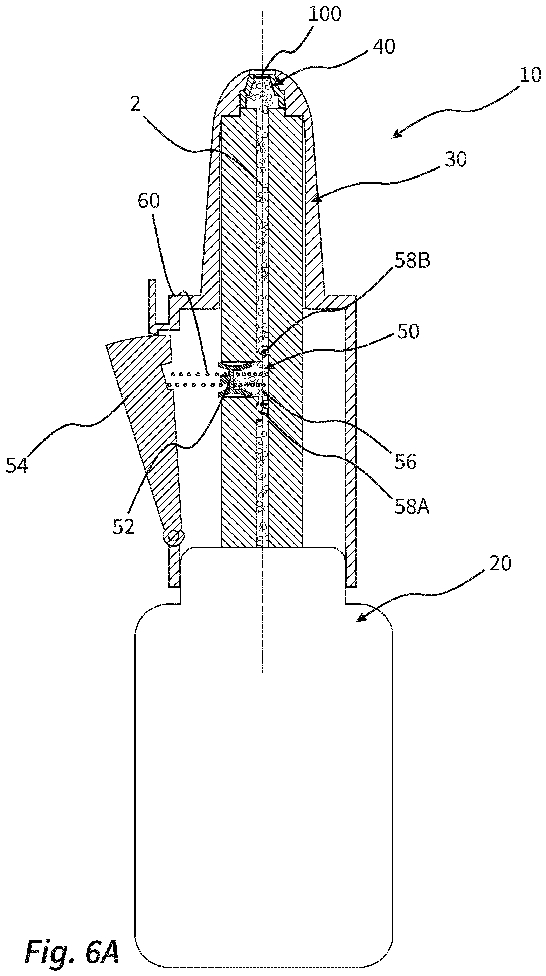

Die Fig. 6A bis 6D zeigen einen zweiten erfindungsgemäßen Flüssigkeitsspender 10, der ebenfalls als Flüssigkeitsspender zur nasalen Applikation von pharmazeutischen Flüssigkeiten vorgesehen ist. Er weist daher ebenfalls einen Nasalapplikator 30 auf, an dessen distalem Ende eine Düseneinheit 40 entsprechend der Fig. 2 bis 4 vorgesehen ist.

Abweichend vom Flüssigkeitsspender der Fig. 1 ist der Flüssigkeitsspender 10 der Fig. 6A bis 6D jedoch als Pumpspender ausgebildet. Seine Fördereinrichtung 50 umfasst daher eine Pumpeinrichtung mit einer Pumpkammer 56, die über einen Einlasskanal mit dem Flüssigkeitsspeicher 20 verbunden ist und die über einen Auslasskanal mit der Düseneinheit 40 verbunden ist. Die Pumpkammer 56 ist durch ein Druckelement 52 in Form eines Kolbens begrenzt. Im Einlasskanal und im Auslasskanal sind jeweils ein druckabhängig öffnendes und schließendes Ventil 58A, 58B vorgesehen, so dass bei einer Pumpkammervergrößerung durch Verlagerung des Druckelements 52 nach links Flüssigkeit aus dem Flüssigkeitsspeicher 20 angesogen wird und durch Verlagerung des Druckelements 52 nach rechts Flüssigkeit aus der Pumpkammer56 in Richtung der Düseneinheit 40 gefördert wird.

Zur Betätigung des Flüssigkeitsspenders der Fig. 6A bis 6D ist eine schwenkbewegliche Betätigungshandhabe 54 an einer seitlichen Fläche des Gehäuses vorgesehen.

Wiederum ist vorgesehen, dass zwischen der Betätigungshandhabe 54 und dem Druckelement 52 ein Federspeicher 60 vorhanden ist.

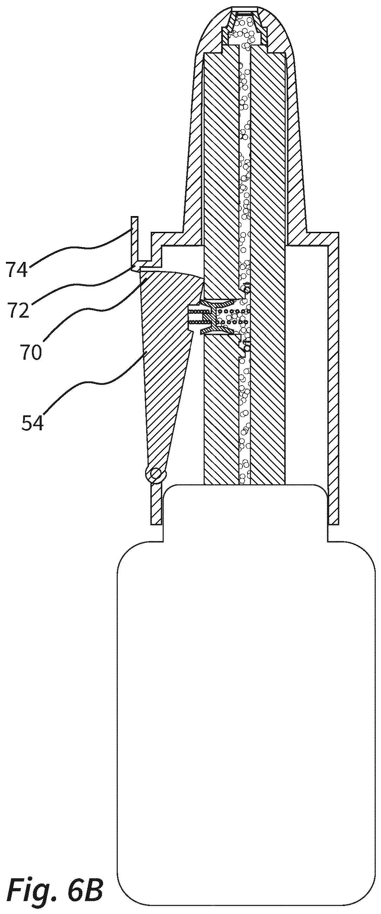

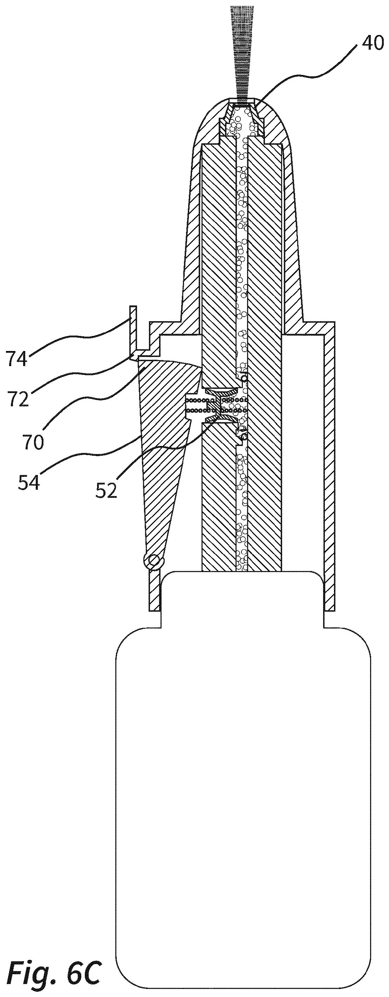

Ausgehend vom Ausgangszustand der Fig. 6A wird bei der Betätigung zunächst die Betätigungshandhabe 54 eingedrückt, so dass sie den Zustand der Fig. 6B einnimmt. In diesem Zustand ist sie durch Rastelemente 70, 72 gehalten, so dass sie zunächst nicht in den Ausgangszustand der Fig. 6A zurückkehren kann. Durch die erfolgte Verlagerung der Betätigungshandhabe 54 in ihre Endlage ist der Federspeicher 60 vollständig komprimiert worden, so dass er ausgehend vom Zustand der Fig. 6B das als Kolben wirkende Druckelemente 52 in Richtung der Pumpkammer 56 verlagert und hierbei deren Volumen verkleinert. Die in der Pumpkammer vorhandene Flüssigkeit wird durch den Austragkanal zur Düseneinheit 40 gefördert und durch die Düsenöffnungen 102 ausgetragen, wie in Fig. 6C dargestellt

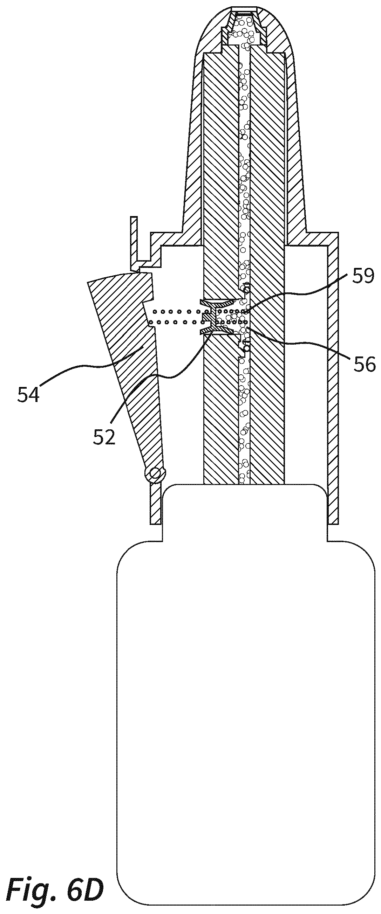

Sobald der Austrag endet, drückt der Patient bestimmungsgemäß auf die Freigabetaste 74, durch die er die Rastelemente 70, 72 voneinander löst, mittels derer die Betätigungshandhabe 54 in ihrer Endlage gehalten wurde. Wie in Fig. 6D dargestellt ist, springt die Betätigungshandhabe 54 durch diese Freigabe zurück in ihre Ausgangslage und eine Rückstellfeder 59 der Pumpeinrichtung drückt das als Kolben agierende Druckelement 52 nach links. Die Pumpkammer 56 vergrößert sich wieder und Flüssigkeit aus dem Flüssigkeitsspeicher 20 wird angesogen.

Damit ist der Ausgangszustand der Fig. 6A wieder hergestellt und ein erneuter Flüssigkeitsaustrag ist durchführbar.

## Patentansprüche

1. Flüssigkeitsspender (10) für nasale Anwendungen mit den folgenden Merkmalen:
a. der Flüssigkeitsspender (10) verfügt über einen Flüssigkeitsspeicher (20), in dem Flüssigkeit vor dem Austrag gelagert ist, und
b. der Flüssigkeitsspender (10) verfügt über einen länglichen Nasalapplikator (30), und
c. an einem distalen Ende des Nasalapplikators (30) ist eine Düsenplatte (100) mit einer Vielzahl von Düsenöffnungen (102) vorgesehen, und
d. die Gesamtheit der Düsenöffnungen (102) ist derart ausgelegt, dass ein Gesamtflüssigkeitsstrom vom mindestens 100 µl / Sekunde durch die Düsenöffnungen (102) erzielt wird, wenn Flüssigkeit mit den Eigenschaften von Wasser in einer der Düsenplatte (100) vorgeschalteten Vorkammer (42) unter einem Druck von 5 bar an der Düsenplatte anliegt.

2. Flüssigkeitsspender nach Anspruch 1 mit dem folgenden weiteren Merkmal:
a. der Gesamtflüssigkeitsstrom durch die Düsenöffnungen (102) beträgt zwischen 100 µl / Sekunde und 750 µl / Sekunde.

3. Flüssigkeitsspender nach Anspruch 2 mit dem folgenden weiteren Merkmal:
a. der Gesamtflüssigkeitsstrom durch die Düsenöffnungen (102) beträgt zwischen 200 µl / Sekunde und 500 µl / Sekunde.

4. Flüssigkeitsspender nach Anspruch 1, 2 oder 3 mit dem folgenden weiteren Merkmal:
a. es sind zwischen 400 und 2000 Düsenöffnungen (102) vorgesehen, wobei die Düsenöffnungen (102) jeweils eine minimale lichte Querschnittsfläche zwischen 10 µm² und 20 µm² aufweisen.

5. Flüssigkeitsspender nach Anspruch 4 mit dem folgenden weiteren Merkmal:
a. die Düsenöffnungen (102) weisen jeweils eine minimale lichte Querschnittsfläche zwischen 10 µm² und 15 µm² auf.

6. Flüssigkeitsspender nach einem der vorstehenden Ansprüchen mit dem folgenden weiteren Merkmal:
a. es sind zwischen 200 und 1000 Düsenöffnungen (102) vorgesehen, wobei die Düsenöffnungen (102) jeweils eine minimale lichte Querschnittsfläche zwischen 20 µm² und 50 µm² aufweisen.

7. Flüssigkeitsspender nach Anspruch 6 mit dem folgenden weiteren Merkmal:
a. die Düsenöffnungen (102) weisen jeweils eine minimale lichte Querschnittsfläche zwischen 25 µm² und 35 µm² auf.

8. Flüssigkeitsspender nach einem der Ansprüche 1 bis 5 mit dem folgenden weiteren Merkmal:
a. es sind zwischen 50 und 250 Düsenöffnungen (102) vorgesehen, wobei die Düsenöffnungen (102) jeweils eine minimale lichte Querschnittsfläche zwischen 50 µm² und 100 µm² aufweisen.

9. Flüssigkeitsspender nach Anspruch 8 mit dem folgenden weiteren Merkmal:
a. die Düsenöffnungen (102) weisen jeweils eine minimale lichte Querschnittsfläche zwischen 70 µm² und 85 µm² auf.

10. Flüssigkeitsspender nach einem der vorstehenden Ansprüche mit den folgenden weiteren Merkmalen:
a. die Düsenplatte (100) ist als metallische Düsenplatte ausgebildet, die durch Galvanoformen hergestellt ist.

11. Flüssigkeitsspender nach einem der Ansprüche 1 bis 9 mit dem folgenden weiteren Merkmal:
a. die Düsenplatte (100) ist als metallische Düsenplatte, als keramische Düsenplatte, als Düsenplatte aus Glas oder einem anderen mineralischen Werkstoff oder als Düsenplatte aus Kunststoff hergestellt.

12. Flüssigkeitsspender nach Anspruch 11 mit dem folgenden weiteren Merkmal:
a. die Düsenöffnungen sind durch Laserbearbeitung eingebracht sind.

13. Flüssigkeitsspender nach einem der Ansprüche 1 bis 9 mit dem folgenden weiteren Merkmal:
a. die Düsenplatte (100) ist aus Kunststoff gefertigt, wobei die Düsenöffnungen im Zuge des Spritzgießens durch die Formgebung der dabei verwendeten Kavität eingebracht sind.

14. Flüssigkeitsspender nach einem der vorstehenden Ansprüche mit dem folgenden weiteren Merkmal:
a. die Düsenöffnungen (102) weisen eine divergierende Ausrichtung auf, insbesondere mit dem zusätzlichen Merkmal:
b. die Düsenplatte ist konvex gewölbt.

## Claims

1. Liquid dispenser (10) for nasal applications, with the following features:
a. the liquid dispenser (10) has a liquid reservoir (20) in which liquid is stored prior to discharge, and
b. the liquid dispenser (10) has an elongated nasal applicator (30), and
c. a nozzle plate (100) with a plurality of nozzle openings (102) is provided at a distal end of the nasal applicator (30), and
d. all the nozzle openings (102) are designed in such a manner that a total liquid flow of at least 100 µl/second through the nozzle openings (102) is achieved if liquid with the properties of water in an antechamber (42) arranged upstream of the nozzle plate (100) under a pressure of 5 bar bears against the nozzle plate.

2. Liquid dispenser according to Claim 1, with the following further feature:
a. the total liquid flow through the nozzle openings (102) is between 100 µl/second and 750 µl/second.

3. Liquid dispenser according to Claim 2, with the following further feature:
a. the total liquid flow through the nozzle openings (102) is between 200 µl/second and 500 µl/second.

4. Liquid dispenser according to Claim 1, 2 or 3, with the following further feature:
a. between 400 and 2000 nozzle openings (102) are provided, wherein the nozzle openings (102) have in each case a minimum clear cross-sectional surface area between 10 µm² and 20 µm².

5. Liquid dispenser according to Claim 4, with the following further feature:
a. the nozzle openings (102) have in each case a minimum clear cross-sectional surface area between 10 µm² and 15 µm².

6. Liquid dispenser according to any one of the preceding claims, with the following further feature:
a. between 200 and 1000 nozzle openings (102) are provided, wherein the nozzle openings (102) have in each case a minimum clear cross-sectional surface area between 20 µm² and 50 µm².

7. Liquid dispenser according to Claim 6, with the following further feature:
a. the nozzle openings (102) have in each case a minimum clear cross-sectional surface area between 25 µm² and 35 µm².

8. Liquid dispenser according to any one of Claims 1 to 5, with the following further feature:
a. between 50 and 250 nozzle openings (102) are provided, wherein the nozzle openings (102) have in each case a minimum clear cross-sectional surface area between 50 µm² and 100 µm².

9. Liquid dispenser according to Claim 8, with the following further feature:
a. the nozzle openings (102) have in each case a minimum clear cross-sectional surface area between 70 µm² and 85 µm².

10. Liquid dispenser according to any one of the preceding claims, with the following further features:
a. the nozzle plate (100) is formed as a metallic nozzle plate, which is produced by electroforming.

11. Liquid dispenser according to any one of Claims 1 to 9, with the following further feature:
a. the nozzle plate (100) is produced as a metallic nozzle plate, as a ceramic nozzle plate, as a nozzle plate composed of glass or another mineral material or as a nozzle plate composed of plastic.

12. Liquid dispenser according to Claim 11, with the following further feature:
a. the nozzle openings are incorporated by laser machining.

13. Liquid dispenser according to any one of Claims 1 to 9, with the following further feature:
a. the nozzle plate (100) is manufactured from plastic, wherein the nozzle openings are incorporated in the course of injection moulding through the shaping of the cavity used in this case.

14. Liquid dispenser according to any one of the preceding claims, with the following further feature:
a. the nozzle openings (102) have a diverging orientation,
in particular with the additional feature:
b. the nozzle plate is convexly arched.

## Revendications

1. Distributeur de liquide (10) pour applications nasales, ayant les caractéristiques suivantes :
a. le distributeur de liquide (10) dispose d'un réservoir de liquide (20) dans lequel le liquide est stocké avant la distribution, et
b. le distributeur de liquide (10) dispose d'un applicateur nasal allongé (30), et
c. une plaque à buses (100) comportant une pluralité d'ouvertures de buses (102) est prévue à une extrémité distale de l'applicateur nasal (30), et
d. l'ensemble des ouvertures de buses (102) est conçu de telle sorte qu'un débit total de liquide d'au moins 100 µl/seconde soit obtenu à travers les ouvertures de buses (102) lorsque du liquide ayant les propriétés de l'eau est appliqué à la plaque à buses dans une préchambre (42) en amont de la plaque à buses (100), sous une pression de 5 bars.

2. Distributeur de liquide selon la revendication 1, avec la caractéristique supplémentaire suivante :
a. le débit total de liquide à travers les ouvertures de buses (102) est compris entre 100 µl/seconde et 750 µl/seconde.

3. Distributeur de liquide selon la revendication 2, avec la caractéristique supplémentaire suivante :
a. le débit total de liquide à travers les ouvertures de buses (102) est compris entre 200 µl/seconde et 500 µl/seconde.

4. Distributeur de liquide selon la revendication 1, 2 ou 3, avec la caractéristique supplémentaire suivante :
a. il est prévu entre 400 et 2000 ouvertures de buses (102), les ouvertures de buses (102) présentant chacune une surface de section transversale libre minimale comprise entre 10 µm² et 20 µm².

5. Distributeur de liquide selon la revendication 4, avec la caractéristique supplémentaire suivante :
a. les ouvertures de buses (102) présentent chacune une surface de section transversale libre minimale comprise entre 10 µm² et 15 µm².

6. Distributeur de liquide selon l'une des revendications précédentes, ayant la caractéristique supplémentaire suivante :
a. il est prévu entre 200 et 1000 ouvertures de buses (102), les ouvertures de buses (102) présentant chacune une surface de section transversale libre minimale comprise entre 20 µm² et 50 µm².

7. Distributeur de liquide selon la revendication 6, avec la caractéristique supplémentaire suivante :
a. les ouvertures de buses (102) présentent chacune une surface de section transversale libre minimale comprise entre 25 µm² et 35 µm².

8. Distributeur de liquide selon l'une des revendications 1 à 5, ayant la caractéristique supplémentaire suivante :
a. il est prévu entre 50 et 250 ouvertures de buses (102), les ouvertures de buses (102) présentant chacune une surface de section transversale libre minimale comprise entre 50 µm² et 100 µm².

9. Distributeur de liquide selon la revendication 8, avec la caractéristique supplémentaire suivante :
a. les ouvertures de buses (102) présentent chacune une surface de section transversale libre minimale comprise entre 70 µm² et 85 µm².

10. Distributeur de liquide selon l'une des revendications précédentes, ayant les caractéristiques supplémentaires suivantes :
a. la plaque à buses (100) est réalisée sous forme de plaque à buses métallique fabriquée par électroformage.

11. Distributeur de liquide selon l'une des revendications 1 à 9, ayant la caractéristique supplémentaire suivante :
a. la plaque à buses (100) est fabriquée sous forme de plaque à buses métallique, de plaque à buses céramique, de plaque à buses en verre ou en un autre matériau minéral, ou de plaque à buses en matière plastique.

12. Distributeur de liquide selon la revendication 11, avec la caractéristique supplémentaire suivante :
a. les ouvertures des buses sont pratiquées par usinage au laser.

13. Distributeur de liquide selon l'une des revendications 1 à 9, ayant la caractéristique supplémentaire suivante :
a. la plaque à buses (100) est fabriquée en matière plastique, les ouvertures de buses étant pratiquées au cours du moulage par injection grâce à la forme de la cavité utilisée à cet effet.

14. Distributeur de liquide selon l'une des revendications précédentes, ayant la caractéristique supplémentaire suivante :
a. les ouvertures de buse (102) présentent une orientation divergente, notamment ayant la caractéristique supplémentaire :
b. la plaque à buses est bombée de manière convexe.
